# EUROPEAN PATENT SPECIFICATION

(11) **EP 3 241 911 B1**
(45) Date of publication and mention of the grant of the patent: **23.10.2019**
(21) Application number: 17159320.5
(22) Date of filing: 14.05.2012
(51) Int. Cl.: C12Q 1/6883, A61K 31/713, G16B 30/00

(54) **IDENTIFICATION OF THROMBOSIS OR BLEEDING RISK IN AN INDIVIDUAL WITH AND WITHOUT ANTI-PLATELET THERAPY**
IDENTIFIZIERUNG VON THROMBOSE- ODER BLUTUNGSRISIKO BEI EINEM PATIENTEN MIT ODER OHNE THROMBOZYTENHEMMTHERAPIE
IDENTIFICATION DE LA THROMBOSE OU RISQUE DE SAIGNEMENT CHEZ UN INDIVIDU AVEC OU SANS TRAITEMENT ANTIPLAQUETTAIRE

(30) Priority: 14.05.2011 EP 11166142
(43) Date of publication of application: 08.11.2017
(62) Divisional of application: 12727319.1
(73) Proprietor: Devenish Nutrition Limited, Belfast BT1 3BG (GB)
(72) Inventor: STANTON, Alice, Navan, Co. Meath (IE); MCCARTHY, Nina, Dublin 2 (IE)
(74) Representative: FRKelly

(56) References cited:
- WO-A1-2005/030993
- US-A1- 2010 035 983
- Nina Mccarthy: "Cardiovascular Genetics: Candidate Gene, Candidate Pathway and Whole Genome Analyses", , 1 September 2011 (2011-09-01), pages 1-316, XP55409203, Retrieved from the Internet: URL:http://epubs.rcsi.ie/cgi/viewcontent.c gi?article=1124&context=phdtheses [retrieved on 2017-09-22]
- KATHIRESAN SEKAR ET AL: "A genome-wide association study for blood lipid phenotypes in the Framingham Heart Study", BMC MEDICAL GENETICS, BIOMED CENTRAL, LONDON, GB, vol. 8, no. Suppl 1, 19 September 2007 (2007-09-19), page S17, XP021028998, ISSN: 1471-2350, DOI: 10.1186/1471-2350-8-S1-S17
- LI YI-DAN ET AL: "NF-kappa B Transcription Factor p50 Critically Regulates Tissue Factor in Deep Vein Thrombosis", JOURNAL OF BIOLOGICAL CHEMISTRY, vol. 284, no. 7, February 2009 (2009-02), pages 4473-4483, XP002663215, ISSN: 0021-9258

## Description

### Introduction

The invention relates to a method for identifying an individual at increased risk of thrombosis or an atherothrombotic event or a major bleeding event selected from cerebral bleeding and gastrointestinal bleeding, as is claimed. The invention also relates to a method of identifying individuals suitable for treatment with a high dosage of anti-platelet therapy or most likely to gain benefit from (single or dual) anti-platelet therapy in the prevention of (primary or secondary) cardiovascular events, or likely to suffer a significant bleeding complication when undergoing treatment with drugs which influence haemostasis, or likely to suffer a thrombotic complication when prescribed a COX2 antagonist or PPARγ agonist, as is claimed.

Thromboxane is released from activated platelets, and plays a key role in the prevention of excessive bleeding by promoting the formation of platelet plugs (thromboses) when blood vessels are injured. However, platelet thromboses, by blocking arterial blood vessels in the coronary and cerebral circulation, are the final step in most heart attacks and strokes, respectively. Hence one of the most commonly prescribed anti-platelet drugs, aspirin, has its beneficial effects through inhibition of platelet formation of thromboxane. Predictably, the major adverse effect of anti-platelet drugs such as aspirin is excessive bleeding.

Thromboxane, after release from platelets, is rapidly metabolised to an inactive metabolite, 11-dehydro-thromboxane B2 (TxM) which is excreted in the urine. Urinary levels of TxM serve as a measure of thromboxane turnover and thrombotic tendency. Consistent with excessive thromboxane formation or turnover playing a causative role in cardiovascular events, high levels of urinary TxM at baseline, both with and without anti-platelet therapy (aspirin), have recently been shown to predict heart attacks and strokes in a number of large epidemiological studies and clinical trials.

Currently, aspirin and other anti-platelet drugs are widely prescribed so as to provide protection from atherothrombotic events. Bleeding is the most serious adverse effect of long-term aspirin and other anti-platelet therapies, with cerebral bleeding (haemorrhagic stroke, intracranial haemorrhage) and gastrointestinal bleeding considered major events. Hence prescription of anti-platelet therapy is currently guided by the relative risks of atherothrombotic events and bleeding, as outlined below: Secondary Prevention: In individuals where the risk of an atherothrombotic event is large (previous heart attack or ischaemic stroke) the protective benefits of single anti-platelet therapy (most commonly aspirin) have been clearly demonstrated to outweigh bleeding risks (BMJ 2002;324;71).

Furthermore while dual anti-platelet therapy (aspirin and ADP receptor antagonists such as clopidogrel and prasugrel) has been shown to be beneficial in the management of acute myocardial infarction and also in patients undergoing angioplasty, dual antiplatelet therapy in patients with stable cardiovascular disease does not reduce adverse cardiovascular outcomes when compared to aspirin alone, and has been associated with an increased risk of bleeding (N. Engl J Med. 2006;354:1706-1717).

Primary Prevention: In individuals who only have one or a number of cardiovascular risk factors (high BP, smoking, diabetes, high cholesterol or obesity), and have not suffered a morbid event as yet, the situation is not clear-cut. In this group, whilst aspirin does reduce the number of heart attacks and strokes, almost as many significant bleeding events (haemorrhagic strokes and severe life-threatening gastrointestinal bleeds) are caused by aspirin (Lancet 2009;373;1849-60)

WO2005030993 discloses an in vitro method for determining the risk of developing thrombosis by identifying polymorphisms of EPCR gene, wherein the presence of G at position 1651, C at position 3610, A at position 4216, or G at position 6936 is indicative of a higher risk to develop thrombosis.

Currently no assessments of baseline thrombotic tendancy or of bleeding risk with anti-platelet therapy, are established in clinical practice. Accurate quantification of urinary TxM is complex and expensive, and remains a research tool exclusively. The conclusion of a recent comparison of currently available platelet function tests was that predictive accuracies of cardiovascular outcomes were at best modest, and that no test provided accurate prognostic information concerning bleeding risks (JAMA 2010;303:754-762). Additional issues included poor reproducibility, long sample processing time, the need for specialised technicians, and/or expense.

In Ireland, and in most of the developed world, over 50% of those over the age of 45 years have at least 2 major cardiovascular risk factors. The fact that a number of large clinical trials are currently ongoing seeking to identify which of these patients derive more benefit than harm from anti-platelet therapy, demonstrates that this is an important current clinical issue, which is attracting funding from both the pharmaceutical industry and governmental grant authorities. These include the ARRIVE Study (Aspirin to Reduce Risk of Initial Vascular Events - A randomized, double-blind, placebo controlled, multicentre, parallel group study to assess the efficacy (reduction of cardiovascular disease events) and safety of 100 mg enteric-coated acetylsalicyclic acid in 12,000 patients at moderate risk of cardiovascular disease. Bayer Healthcare AG funded study costing at least 36 million Euro) and the Japanese Primary Prevention Project - JPPP (A randomised, open-label, controlled trial of aspirin versus no aspirin in 14,466 patients with multiple risk factors for vascular events. Funded by the Japanese Ministry of Health, Labour and Welfare, and the Waksman Foundation of Japan Inc.)

It is an object of the invention to overcome at least one of the above-referenced problems.

### Statements of Invention

The Applicant has discovered that the presence of certain single nucleotide polymorphisms (SNP) variants as defined in the claims are significantly associated with higher TxM, and the association of TxM with the SNPs is almost totally driven by the patients not taking aspirin, whilst there is little or no statistically significant association between any of these SNPs with TxM among aspirinated subjects.

The SNP variants employed in the present invention are located in the gene CNTN4.

PPARGC1β encodes peroxisome proliferator-activated receptor gamma co-activator 1β. Through formation of multimeric complexes with the nuclear receptor, peroxisome proliferator-activated receptor gamma (PPARγ), PPARGC1β influences transcriptional activity and gene expression in a range of metabolic pathways. Both PPARGC1β and PPARγ are expressed in haematopoietic tissue, but the effects of PPARγ activation on megakaryocyte formation of platelets, or on thrombosis have not been studied.

CNTN4 encodes contactin 4, a member of the contactin family ofimmunoglobulins. Contactins are axon-associated cell adhesion molecules that function in neuronal network formation and plasticity. Contactin 4 is mainly expressed in brain, although lower levels of expression in other tissues has been shown. So far, there are no data suggesting a role for contactin 4 in thrombosis.

LZTS1 encodes leucine zipper, putative tumor suppressor 1, a tumor suppressor protein that is ubiquitously expressed in normal tissues. It is involved in the regulation of cell growth, and may stabilize the active CDC2-cyclin B1 complex and thereby prevent uncontrolled cell proliferation. Again, so far there are no data suggesting a role for this gene in thrombosis.

KCNE4 encodes potassium voltage-gated channel, Isk-related family, member 4. This member is a type I membrane protein, an ancillary protein that assembles as a beta subunit with a voltage-gated potassium channel complex of pore-forming alpha subunits, modulating the gating kinetics and enhancing stability of the multimeric complex. Little is known about the function of this potassium channel specifically; voltage-gated potassium channels have diverse functions including the regulation of neurotransmitter release, heart rate, insulin secretion, neuronal excitability, epithelial electrolyte transport, smooth muscle contraction, and cell volume. This gene is prominently expressed in the embryo and in the adult uterus, and is not known to play a role in thrombosis.

The above findings clearly illustrate that genetic variants in PPARGC1β CNTN4, LZTS1 and KCNE4 strongly influence thromboxane formation - carriage of one of these SNPs was associated with an increment in TxM of similar magnitude to the decrement in TxM associated with the taking of aspirin. Furthermore since the associations were only seen in aspirin naive patients, it appears that aspirin completely or in large part blocks the excess production of thromboxane in carriers of PPARGC1β CNTN4, LZTS1 and KCNE4 genetic variants.

Given the very dramatic difference in urinary TxM according to PPARGC1β, CNTN4, LZTS1 and KCNE4 genotype, identification of PPARGC1β CNTN4, LZTS1 and KCNE4 variants allows identification of subjects at greater and lesser risk of thrombosis and of bleeding. It allows identification of those subjects who will gain greatest benefit from anti-platelet therapy (carriers of pro-thrombotic PPARGC1β CNTN4, LZTS1 and KCNE4 genetic variants), and also those most likely to suffer bleeding complications when prescribed anti-platelet drugs and also other drugs which influence haemostasis such as non steroidal anti-inflammatory drugs and anticoagulants (non-carriers).

Further applications for PPARGC1β, CNTN4, LZTS1, and KCNE4 genotyping include the identification of subjects most likely to suffer thrombotic side effects with widely used drugs such as COX2 inhibitors and glitazones - these hitherto unpredictable adverse events have led to the withdrawal from the market of some block-busters such as oral nimesulide (COX2 inhibitor now only available as a topical gel) and the issueing of a black box warning for rosiglitazone (glitazone). COX2 antagonists inhibit the formation of prostacyclin (natural antagonist of thromboxane) to a greater extent than the formation of thromboxane, and hence use of COX2 antagonists in carriers of pro-thrombotic PPARGC1β CNTN4, LZTS1 and KCNE4 genetic variants would be more likely to lead to thrombotic events. Glitazones such as rosiglitazone are PPARγ agonists, and hence their use in carriers of pro-thrombotic PPARGC1β genetic variants may well be the explanation of excess thrombotic events.

The invention provides a method for identification of an individual at increased risk of thrombosis or an atherothrombotic event, comprising a step of assaying a biological sample from the individual for a minor allelic variant selected from SEQUENCE ID NO's 22 and 25, wherein the presence of a minor allelic variant of SEQUENCE ID NO 22 correlates with the individual being at increased risk of a thrombosis or an atherothrombotic event, and wherein absence of the minor allelic variant of SEQUENCE ID NO 25 correlates with the individual being at increased risk of a thrombosis or an atherothrombotic event.

The invention also provides a method for identification of an individual at increased risk of a major bleeding event selected from cerebral bleding and gastrointestinal bleeding, comprising a step of assaying a biological sample from the individual for a minor allelic variant selected from SEQUENCE ID NO's 22 and 25, wherein the absence of a minor allelic variant of SEQUENCE ID NO 22 correlates with the individual being at increased risk of a major bleeding event, and wherein the presence of the minor allelic variant of SEQUENCE ID NO 25 correlates with the individual being at increased risk of a major bleeding event.
selected from cerebral bleeding and gastrointestinal bleeding.

The invention also provides a method of identifying an individual most likely to gain benefit from single anti-platelet therapy in the primary prevention of cardiovascular events, the method comprising a step of assaying a biological sample from the individual for a minor allelic variant selected from SEQUENCE ID NO's 22 and 25, wherein the presence of a minor allelic variant of SEQUENCE ID NO 22 or absence of the minor allelic variant of SEQUENCE ID NO: 25 correlates with the individual being suitable for single anti-platelet therapy.

The invention also provides a method of identifying an individual suitable for treatment with a high dosage of anti-platelet therapy, the method comprising a step of assaying a biological sample from the individual for a minor allelic variant selected from SEQUENCE ID NO's 22 and 25, wherein the presence of a minor allelic variant of SEQUENCE ID NO 22 or absence of the minor allelic variant of SEQUENCE ID NO: 25 correlates with the individual being suitable for a high dosage of anti-platelet therapy.

The invention also provides a method of identifying an individual most likely to gain benefit from dual anti-platelet therapy in the secondary prevention of cardiovascular events, the method comprising a step of assaying a biological sample from the individual for a minor allelic variant selected from SEQUENCE ID NO's 22 and 25 wherein the presence of a minor allelic variant of SEQUENCE ID NO 22 or absence of the minor allelic variant of SEQUENCE ID NO: 25 correlates with the individual being suitable for dual anti-platelet therapy.

The invention also provides a method of identifying an individual likely to suffer a significant bleeding complication when undergoing treatment with drugs which influence haemostasis, the method comprising a step of assaying a biological sample from the individual for a minor allelic variant selected from SEQUENCE ID NO's 22 and 25 wherein the absence of a minor allelic variants of SEQUENCE ID NO 22 or presence of the minor allelic variant of SEQUENCE ID NO: 25 correlates with the individual being likely to suffer a significant bleeding complication when undergoing treatment with a drug that influences haemostasis.

The invention also provides a method of identifying an individual likely to suffer a thrombotic complication when prescribed a COX2 antagonist or PPARy agonist, the method comprising a step of assaying a biological sample from the individual for a minor allelic variant selected from SEQUENCE ID NO's 22 and 25, wherein the presence of a minor allelic variant of SEQUENCE ID NO's 22 or absence of the minor allelic variant of SEQUENCE ID NO: 25 correlates with the individual being likely to suffer a thrombotic complication when prescribed a COX2 antagonist or a PPARy agonist.

Also disclosed is a method for prevention or treatment of thrombosis or an atherothrombotic event in an individual, which method employs a step of identifying an individual at increased risk of thrombosis or an atherothrombotic event according to a method of Claim 1, wherein an individual identified as being at increased risk of thrombosis or an atherothrombotic event is treated with an anti-platelet agent.

Also disclosed is a method for prevention of a major bleeding event in an individual, which method employs a step of identifying an individual at increased risk of having a major bleeding event according to a method of Claim 2, wherein an individual identified as being at increased risk of having a major bleeding event is removed from anti-platelet therapy.

Also disclosed is a method for primary prevention of a cardiovascular event in an individual, which method employs a step of identifying an individual most likely to gain benefit from single anti-platelet therapy in the primary prevention of cardiovascular events according to Claim 3, wherein identified individual is treated with a single anti-platelet agent.

Also disclosed is a method of treatment of an individual with anti-platelet therapy in the secondary prevention of cardiovascular events, which method employs a step of identifying an individual suitable for treatment with a high dosage of anti-platelet therapy according to a method of Claim 4, wherein an identified individual is treated with a high dose of anti-platelet agent.

Also disclosed is a method for secondary prevention of a cardiovascular event in an individual, which method employs a step of identifying an individual most likely to gain benefit from dual anti-platelet therapy in the secondary prevention of cardiovascular events according to a method of Claim 5, wherein an identified individual is treated with a dual anti-platelet therapy.

The methods, assay and systems of the invention employ 2 variants The methods, assays or systems of the invention employ 2 minor allelic variants selected from SEQUENCE ID NO:s 22 and 25. The 2 minor allelic variants selected from SEQUENCE ID NO:s 22 and 25 are employed. Generally, use of two or more variants in the methods/assays and systems of the invention provide for a more powerful diagnosis.

Also disclosed is a computer program comprising program instructions for causing a computer to perform the method of the invention.

The invention also provides an assay for identifying a subject as having an increased probability of suffering a thrombotic or atherothrombotic event comprising: (a) identifying at least one allele of a single nucleotide polymorphism selected from the group consisting of SEQUENCE ID NO'S 22 and 25 in a biological sample obtained from a subject; and (b) comparing the at least one identified allele to a list of corresponding high risk alleles selected from the minor allelic variants of SEQUENCE ID NO 22 and the major allelic variant of SEQUENCE ID NO 25, and if the biological sample comprises one or more of the risk alleles then identifying the subject as having an increased probability of suffering a thrombotic or atherothrombotic event.

The invention also provides an assay for identifying a subject having an increased probability of suffering a major bleeding event comprising: (a) identifying at least one allele of a single nucleotide polymorphism selected from the group consisting of SEQUENCE ID NO's 22 and 25 in a biological sample obtained from a subject who is at increased risk of a major bleeding event; and (b) comparing the at least one identified allele to a list of corresponding high risk alleles selected from the major allelic variants of SEQUENCE ID NO 22 and the minor allelic variant of SEQUENCE ID NO 25, and if the biological sample comprises one or more of the risk alleles then identifying the subject as having an increased probability of suffering a major bleeding event.

The invention also provides an assay for selecting a primary or secondary treatment regimen for a subject at increased risk of thrombosis or atherothrombotic event comprising: (a) subjecting a test sample of the subject to at least one analysis to determine the alleles of a single nucleotide polymorphism selected from the group consisting of SEQUENCE ID NO's 22 and 25 and (b) comparing the at least one identified allele to a list of corresponding high risk alleles selected from the minor allelic variants of SEQUENCE ID NO 22 and the major allelic variant of SEQUENCE ID NO 25, and if the biological sample comprises one or more of the risk alleles then selecting a single anti-platelet therapy as a treatment regime in the primary prevention of a cardiovascular event or dual anti-platelet therapy as a treatment regime in the secondary prevention of a cardiovascular event.

The invention also provides an assay for selecting a treatment regime for an individual at increased risk of thrombosis or an atherothrombotic event, the assay comprising: (a) subjecting a test sample of the subject to at least one analysis to determine the alleles of a single nucleotide polymorphism selected from the group consisting of SEQUENCE ID NO's 22 and 25 and (b) comparing the at least one identified allele to a list of corresponding high risk alleles selected from the minor allelic variants of SEQUENCE ID NO 22 and the major allelic variant of SEQUENCE ID NO 25, and if the biological sample comprises one or more of the risk alleles then selecting a high dosage of anti-platelet therapy.

The invention also provides an assay for selecting a treatment regime for an individual undergoing treatment with a drug that influences haemostasis: (a) subjecting a test sample of the subject to at least one analysis to determine the alleles of a single nucleotide polymorphism selected from the group consisting of SEQUENCE ID NO's 22 and 25 and (b) comparing the at least one identified allele to a list of corresponding high risk alleles selected from the major allelic variants of SEQUENCE ID NO 22 24 and the minor allelic variant of SEQUENCE ID NO 25, and if the biological sample comprises one or more of the risk alleles then selecting a treatment that does not include an anti-platelet agent.

In the assays of the invention, the step of comparing is preferably performed with a non-human machine, for example a computer.

Also disclosed is a system for obtaining data from at least one test sample obtained from at least one individual, the system comprising:
- a determination module configured to receive at least one test sample and perform at least one test analysis on the test sample to determine the presence or absence of one or more minor allelic variants of SEQUENCE ID NO:'s 22 and 25
- optionally, a storage system for storing allelic variant data generated by the determination system; and
- a display module for displaying a content based in part on the data output from said determination module, wherein the content comprises a signal indicative of the presence or absence of at least one of the minor allelic variants of SEQUENCE ID NO's 22 and 25.

Typically, the system is for identifying an individual's risk of thrombosis or an atherothrombotic event, in which the system comprises a correlation module for correlating allelic variant data from the determination module with risk of thrombosis or an atherothrombotic event, wherein the presence of a minor allelic variant of SEQUENCE ID NO 22 or absence of a minor allelic variant of SEQUENCE ID NO 25 correlates with the individual being at increased risk of thrombosis or an atherothrombotic event, and wherein the display module displays a content based in part on the data from the correlation system, the content optionally comprising a signal indicative of the individuals risk of thrombosis or an atherothrombotic event.

Typically, the system is for identifying an individual's risk of a major bleeding event, in which the system comprises a correlation module for correlating allelic variant data from the determination module with risk of a major bleeding event, wherein the absence of a minor allelic variants of SEQUENCE ID NO 22 or presence of a minor allelic variant of SEQUENCE ID NO 25 correlates with the individual being at increased risk of a major bleeding event, and wherein the display module displays a content based in part on the data from the correlation system, the content optionally comprising a signal indicative of the individuals risk of a major bleeding event.

Typically, the system is for performing a method for identification of an individual most likely to gain benefit from single anti-platelet therapy in the primary prevention of cardiovascular events or dual anti-platelet therapy in the secondary treatment of cardiovascular events, in which the system comprises a correlation module for correlating allelic variant data from the determination module with likelihood of the individual gaining benefit from single anti-platelet therapy or dual anti-platelet therapy, wherein the presence of a minor allelic variant of SEQUENCE ID NO 22 or absence of a minor allelic variant of SEQUENCE ID NO 25 correlates with a likelihood of the individual gaining benefit from single anti-platelet therapy in the primary prevention of cardiovascular events or dual anti-platelet therapy in the secondary treatment of cardiovascular events, and wherein the display module displays a content based in part on the data from the correlation system, the content optionally comprising a signal indicative of the likelihood of the individual gain benefit from single anti-platelet therapy in the primary prevention of cardiovascular events or dual anti-platelet therapy in the secondary treatment of cardiovascular events.

Typically, the system is for identifying a treatment regime for an individual at risk of thrombosis or an atherothrombotic event, in which the system comprises a correlation module for correlating allelic variant data from the determination module with a suitable treatment regime, wherein the presence of a minor allelic variant of SEQUENCE ID NO 22 or absence of a minor allelic variant of SEQUENCE ID NO 25 correlates with the individual being suitable for treatment with a high dose of anti-platelet therapy, and wherein the display module displays a content based in part on the data from the correlation system, the content optionally comprising a signal indicative of a treatment regime suitable for the individual.

Typically, the system is for identification of an individual at risk of suffering a significant bleeding complication when undergoing treatment with drugs which influence haemostasis, in which the system comprises a correlation module for correlating allelic variant data from the determination module with risk of a significant bleeding event, wherein the absence of a minor allelic variants of SEQUENCE ID NO 22 or presence of a minor allelic variant of SEQUENCE ID NO 25 correlates with the individual being at increased risk of a significant bleeding complication, and wherein the display module displays a content based in part on the data from the correlation system, the content optionally comprising a signal indicative of the individuals risk of suffering a significant bleeding complication when undergoing treatment with drugs which influence haemostasis.

Typically, the system is for identification of risk of an individual suffering a thrombotic complication when prescribed a COX2 antagonist or PPARγ agonist, in which the system comprises a correlation module for correlating allelic variant data from the determination module with risk of a thrombotic complication, wherein the presence of a minor allelic variant of SEQUENCE ID NO's 22 and 25 or absence of a minor allelic variant of SEQUENCE ID NO 25 correlates with risk of a thrombotic complication, and wherein the display module displays a content based in part on the data from the correlation system, the content optionally comprising a signal indicative of risk of the individual suffering a thrombotic complication.

Ideally, the determination system comprises a PCR apparatus.

Also disclosed is a PPARGC1β antagonist for use in the treatment or prevention of an arterial thrombotic or bleeding disorder.

Also disclosed is a PPARGC1β agonist for use in the treatment or prevention of an arterial thrombotic or bleeding disorder.

Preferably, the PPARGC1β antagonist is an siRNA molecule.

Also disclosed is a kit or device capable of identifying an allelic variant selected from the single nucleotide polymorphisms of SEQUENCE ID NO's 22 and 25 for use in identification of an individual at increased risk of thrombosis or an atherothrombotic event, or a major bleeding event

Also disclosed is a kit or device capable of identifying of identifying an allelic variant selected from the single nucleotide polymorphisms of SEQUENCE ID NO's 22 and 25 for use in (a) identifying an individual suitable for treatment by anti-platelet therapy (b) a suitable treatment regime for an individual at risk of thrombosis or an atherothrombotic event.

### Detailed Description of the Invention

The invention relates to an assay/method/system for identifying individuals that have a tendancy to thrombotic, atherothrombotic or bleeding events or an assay/method/system for identifying suitable treatment regimes for individuals in need thereof as is claimed. The diagnostic/prognostic variable is one of a group of SNP variants identified in the claims, located in the CNTN4, gene. The identification of such individuals is useful as it allows a clinician to tailor clinical intervention based on the risk identified by the assay. The invention also relates to a pharmacogenomic assay for identifying individuals who are suitable for anti-platelet therapy, in which the biomarker is one of a group of SNP variables identified in the claims, located in the CNTN4, gene. The assay is useful as it helps identify individuals for which the antithrombotic benefits of the anti-platelet drug outweigh the bleeding risks.

The details of SNP's are provided in Tables 1 and 2 below, and the attached sequence listing, as follows:
- SEQ ID NO: 1 (rs4235745) is a C→T change located at position 149171304 of the PPARGC1β gene, in which T is the minor allele.
- SEQ ID NO: 2 (rs32582) is a C→A change located at position 149185610 of the PPARGC1β gene, in which A is the minor allele.
- SEQ ID NO: 3 (rs28282) is a C→G change located at position 149182399 of the PPARGC1β gene, in which G is the minor allele.
- SEQ ID NO: 4 (rs32581) is a G→T change located at position 149185823 of the PPARGC1β gene, in which T is the minor allele.
- SEQ ID NO: 5 (rs32574) is a C→A change located at position 149199079 of the PPARGC1β gene, in which A is the minor allele.
- SEQ ID NO: 6 (rs17110447) is a A→G change located at position 149173039 of the PPARGC1β gene, in which G is the minor allele.
- SEQ ID NO: 7 (rs2161257) is a A→G change located at position 149170190 of the PPARGC1β gene, in which G is the minor allele.
- SEQ ID NO: 8 (rs32579) is a C→T change located at position 149191041 of the PPARGC1β gene, in which T is the minor allele.
- SEQ ID NO: 9 (rs10515638) is a G→T change located at position 149132724 of the PPARGC1β gene, in which T is the minor allele.
- SEQ ID NO: 10 (rs32586) is a A→G change located at position 149181113 of the PPARGC1β gene, in which G is the minor allele.
- SEQ ID NO: 11 (rs32588) is a T→C change located at position 1491180236 of the PPARGC1β gene, in which C is the minor allele.
- SEQ ID NO: 12 (rs32589) is a G→A change located at position 149180082 of the PPARGC1β gene, in which A is the minor allele.
- SEQ ID NO: 13 (rs251464) is a G→C change located at position 149176427 of the PPARGC1β gene, in which C is the minor allele.
- SEQ ID NO: 14 (rs251468) is a C→T change located at position 149174678 of the PPARGC1β gene, in which T is the minor allele.
- SEQ ID NO: 15 (rs17110375) is a A→G change located at position 149104421 of the PPARGC1β gene, in which G is the minor allele.
- SEQ ID NO: 16 (rs251460) is a T→C change located at position 149177423 of the PPARGC1β gene, in which C is the minor allele.
- SEQ ID NO: 17 (rs251463) is a G→A change located at position 149176522 of the PPARGC1β gene, in which A is the minor allele.
- SEQ ID NO: 18 (rs2052490) is a C→A change located at position 149158366 of the PPARGC1β gene, in which A is the minor allele.
- SEQ ID NO: 19 (rs12189417) is a G→C change located at position 149168322 of the PPARGC1β gene, in which C is the minor allele.
- SEQ ID NO: 20 (rs32577) is a G→A change located at position 149192993 of the PPARGC1β gene, in which A is the minor allele.
- SEQ ID NO: 21 (rs741580) is a A→G change located at position 149181863 of the PPARGC1β gene, in which G is the minor allele.
- SEQ ID NO: 22 (rs10510230) is a A→C change located at position 2257130 of the CNTN4 gene, in which C is the minor allele.
- SEQ ID NO:23 (rs10503687) is a A→G change located at position 20628221 of the LZTS1 gene, in which G is the minor allele.
- SEQ ID NO: 24 (rs10190010) is a A→T change located at position 223714451 of the KCNE4 gene, in which T is the minor allele.
- SEQ ID NO: 25 (rs4684343) is a G→A change located at position 2366098 of the CNTN4 gene, in which A is the minor allele.

In this specification, the term "thrombosis" refers to the formation of a blood clot inside a blood vessel which obstructs the blood flow within the vessel. The term "atherothrombotic event" refers to an event caused by blockage of an artery by a thrombus, resulting in local obstruction of blood flow leading to hypoxic or anoxic conditions. Examples of atherothrombotic events include stroke and heart attack.

In this specification, the terms "major bleeding event" and "significant bleeding complication" are art-recognised terms which would be understood by a preventative cardiologist. The terms should be understood to include bleeding events such as cerebral bleeding (such as haemorrhagic stroke or intracranial haemorrhage) or gastrointestinal bleeding.

In this specification, the term "dual anti-platelet therapy" should be understood to mean treatment with aspirin (or an aspirin variant/analogue) and a second anti platelet agent such as an ADP receptor antagonist (i.e. clopidogrel, ticlopidine or prasugrel), or dipyridamole.

The term "high dosage" as applied to anti-platelet agents should be understood to mean a dosage that is higher than that conventionally prescribed for patients. Thus, for aspirin or clopidogrel for example, a high dosage means a dosage higher than 100mg daily, for example a dosage of 150-300mg or higher.

In this specification, the term "primary prevention of cardiovascular events" should be understood to mean treatment of individuals who only have one or a number of cardiovascular risk factors (high BP, smoking, diabetes, high cholesterol or obesity), and have not suffered a morbid event as yet. In this group, whilst aspirin does reduce the number of heart attacks and strokes, almost as many significant bleeding events (haemorrhagic strokes and severe life-threatening gastrointestinal bleeds) are caused by aspirin (Lancet 2009;373;1849-60)

In this specification, the term "secondary treatment of cardiovascular events" refers to treatment of individuals where the risk of an atherothrombotic event is large (previous heart attack or ischaemic stroke) and the protective benefits of single anti-platelet therapy (most commonly aspirin) have been clearly demonstrated to outweigh bleeding risks (BMJ 2002;324;71).

In this specification, the term "drugs which influence haemostasis" should be understood to mean anti-platelet drugs, anti-coagulants, non-steroidal anti-inflammatory drugs, and fibrinolytics/thrombolytics.

In this specification, the term "thrombotic disorder" should be understood to mean increased risk of formation of pathological intravascular thromboses

In this specification, the term "bleeding disorder" should be understood to mean increased likelihood of pathological bleeding events occurring.

The diagnostic variable of the invention is an allelic variant of SEQUENCE ID NO's 22 and 25. Thus, carriage of a minor allelic variant of SEQUENCE ID NO 22 or a major allelic variant of SEQUENCE ID NO 25 indicates risk of thrombosis or an atherothrombotic event. Likewise, absence of any of these variants would indicate low risk of thrombosis or an atherothrombosis. Further, absence of any of the minor allelic variants of SEQUENCE ID NO 22 or a major allelic variant of SEQUENCE ID NO 25 indicates risk of a major bleeding event in an individual, especially an individual on anti-platelet therapy.

The methods of the invention are applicable to individuals who are healthy as well as individuals with established cardiovascular disease or individuals deemed to be at risk of having an atherothrombotic event such as a heart attack or a stroke or a major bleeding event. Suitably, the methods of the invention are for individuals that are deemed by a clinician to be at risk of having thrombosis or an atherothrombotic event.

The term "anti-platelet therapy" as used herein refers to treatment of an individual with a platelet antagonist with the aim of attenuating platelet activity. Examples of anti-platelet agents include COX-1 inhibitors (i.e. aspirin and aspirin analogues), ADP receptor inhibitors, adenosine reuptake inhibitors, and the like. The term "single anti-platelet therapy" means treatment with a single platelet antagonist.

The term "aspirin analogue" as used herein refers to variants of aspirin (acetylsalicyclate) that retain platelet COX-1 inhibitory activity. Examples of aspirin analogues in the literature include Alagha et al. (Bioorganic & Medicinal Chemistry Letters, Vol. 19, Issue 15, August 2009), The term "aspirin therapy" refers to treatment with aspirin or an aspirin analogue.

For an individual positive for the minor allelic variants of SEQ ID NO 22 the terms "increased risk of thrombosis" or "increased risk of an atherothrombotic event" should be understood to mean an increased risk of thrombosis (typically arterial thrombosis) or an atherothrombotic event compared to an individual who does not carry the minor allelic variant of SEQ ID NO 22. For an individual negative for the minor allelic variant of SEQ ID NO 25, the term "increased risk of thrombosis" or "increased risk of an atherothrombotic event" should be understood to mean an increased risk of thrombosis (typically arterial thrombosis) or an atherothrombotic event compared to an individual who is positive for the minor allelic variant of SEQ ID NO 25.

For an individual negative for the minor allelic variant of SEQ ID NO 22 the term "increased risk of a major bleeding event" should be understood to mean that the individual is more likely to suffer a major bleeding event compared to an individual who carried the minor allelic variants of SEQ ID NO 22. For an individual positive for the minor allelic variant of SEQ ID NO 25, the term "increased risk of a major bleeding event" should be understood to mean that the individual is more likely to suffer a major bleeding event, compared to an individual who is negative for the minor allelic variant of SEQ ID NO 25.

The term "arterial thrombosis" should be understood to mean thrombosis that is predominantly mediated by platelet adhesion and aggregation, and which is the main cause of most heart attacks and strokes, and is distinct from venous thrombosis which is a form of thrombosis predominantly mediated by the clotting cascade.

The term "biological sample" refers to any biological material from the individual for example blood, serum, urine, saliva, tissue, cerebrospinal fluid and the like. Typically, the biological sample is, or is obtained from, blood, for example as blood cell, preparation, or lymphocyte, preparation obtained from the individuals blood.

In this specification, the term "treating" refers to administering a medicament, for example one or more anti-platelet agents (or in certain cases a PPARGC1β agonist or antagonist) to an individual that has, or is likely or predisposed to develop, a thrombotic, atherothrombotic, or a bleeding disorder with the purpose to cure, heal, prevent, alleviate, relieve, alter, remedy, ameliorate, or improve the thrombotic or bleeding disorder. The term "therapeutically effective amount" refers to the amount of the medicament, for example an anti-platelet agent or in some cases a PPARGC1β agonist or antagonist that is required to confer the intended therapeutic effect in the individual, which amount will vary depending on the type of medicament, route of administration, type and status of the thrombotic or bleeding disorder, and possible inclusion of other therapeutics or excipients.

To practice the methods, the above-described pharmaceutical composition can be administered orally, parenterally, by inhalation spray, topically, rectally, nasally, bucally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. A sterile injectable composition, e.g., a sterile injectable aqueous or oleaginous suspension, can be formulated according to techniques known in the art using suitable dispersing or wetting agents (such as Tween 80) and suspending agents. The sterile injectable preparation can also be a sterile injectable solution or suspension in a non-toxic parentally acceptable diluents or solvent for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (eg. Synthetic mono-or dyglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluents or dispersant, or carboxymethyl cellulose or similar dispersing agents. Other commonly used surfactants such as Tweens or Spans or other similar emulsifying agents or bioavailablity enhancers which are commonly used in the manufacture of pharmaceutically acceptable solid, liquid, or other dosage forms can also be used for the purposes of formulation.

A composition for oral administration can be any orally acceptable dosage form including, but not limited to, capsules, tablets, emulsions and aqueous suspensions, dispersions and solutions. In the case of tablets for oral use, carriers that are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavouring, or colouring agents can be added. A nasal aerosol or inhalation composition can be prepared according to techniques well known in the art of pharmaceutical formulation. A fused multicyclic compound-containing composition can also be administered in the form of suppositories for rectal administration.

The carrier in the pharmaceutical composition must be "acceptable" in the sense of being compatible with the active ingredient of the formulation (and preferable, capable of stabilising it) and not deleterious to the subject to be treated. For example, one or more solubilising agents, which form more soluble complexes with the fused multicyclic compounds, or more solubilising agents, can be utilised as pharmaceutical carriers for delivery of the active compounds. Examples of other carriers include colloidal silicon dioxide, magnesium stearate, sodium lauryl sulphate, and D&C Yellow #10.

The term "PPARGC1β antagonist/agonist" refers to a compound that is capable of decreasing/increasing the activity of PPARGC1β *in vivo,* respectively. The activity may be decreased in a number of different ways which will be apparent to a person skilled in the art, including reducing the expression of the protein (for example by means of low molecular weight antagonist/inhibitors such as for example siRNA or shRNA), or by directly inhibiting the activity of the protein by administering a chemical inhibitor or an antibody that has specific binding affinity for PPARGC1β or a PPARGC1β subunit. In a preferred embodiment of the invention, the invention relates to a low molecular weight antagonist/inhibitor of PPARGC1β expression, the details of which will be well known to the person skilled in the field of molecular biology, and which include siRNA, shRNA, miRNA, antisense oligonucleotides, and ribozyme molecules. Micro RNA's (miRNAs) are small (∼22nt) non-coding RNAs (ncRNAs) that regulate gene expression at the level of translation. Alternatively, small hairpin RNA (shRNA) molecules are short RNA molecules having a small hairpin loop in their tertiary structure that may be employed to silence genes. The design of miRNA or shRNA molecules capable of silencing PPARGC1β will be apparent to those skilled in the field of miRNA or shRNA molecule design. As an alternative, the level of PPARGC1β expression can be modulated using antisense or ribozyme approaches to inhibit or prevent translation of PPARGC1β mRNA transcripts or triple helix approaches to inhibit transcription of the PPARGC1β gene. Antisense approaches involve the design of oligonucleotides (either DNA or RNA) that are complementary to PPARGC1β mRNA. The antisense oligonucleotides will bind to the complementary mRNA transcripts and prevent translation. Ribozyme molecules designed to catalytically cleave PPARGC1β mRNA transcripts can also be used to prevent translation and expression of PPARGC1β. (See, e.g., PCT International PublicationWO90/11364, published October 4,1990 ; Sarver et al. , 1990, Science 247: 1222-1225).

In one embodiment of the invention, the PPARGC1β antagonist/inhibitor is a PPARGC1β antagonist. One example of a PPARGC1β antagonist is an anti-PPARGC1β antibody (i.e. an antibody which specifically binds to human PPARGC1β protein). An example of such an antibody is an anti- PPARGC1β polyclonal antibody sold by Abnova under catalogue number H00133522-A01. In particular, purified PPARGC1β may be used to produce antibodies or to screen libraries of pharmaceutical agents to identify those which specifically bind PPARGC1β. Antibodies to PPARGC1β may also be generated using methods that are well known in the art. Such antibodies may include, but are not limited to, polyclonal, monoclonal, chimeric, and single chain antibodies, Fab fragments, and fragments produced by a Fab expression library. Neutralizing antibodies (i.e., those which inhibit dimer formation) are generally preferred for therapeutic use. Single chain antibodies (e.g., from camels or llamas) may be potent enzyme inhibitors and may have advantages in the design of peptide mimetics, and in the development of immuno-adsorbents and biosensors (Muyldermans, S. (2001) J. Biotechnol. 74:277-302). For the production of antibodies, various hosts including goats, rabbits, rats, mice, camels, dromedaries, llamas, humans, and others may be immunized by injection with PPARGC1β or with any fragment or oligopeptide thereof which has immunogenic properties (especially the fragment specified above). Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, KLH, and dinitrophenol.

It is preferred that the oligopeptides, peptides, or fragments used to induce antibodies to PPARGC1β have an amino acid sequence consisting of at least about 5 amino acids, and generally will consist of at least about 10 amino acids. It is also preferable that these oligopeptides, peptides, or fragments are identical to a portion of the amino acid sequence of the natural protein. Monoclonal antibodies to PPARGC1β may be prepared using any technique which provides for the production of antibody molecules by continuous cell lines in culture. These include, but are not limited to, the hybridoma technique, the human B-cell hybridoma technique, and the EBV-hybridoma technique. (See, e.g., Kohler, G. et al. (1975) Nature 256:495-497; Kozbor, D. et al. (1985) J. Immunol. Methods 81:31-42; Cote, R. J. et al. (1983) Proc. Natl. Acad. Sci. USA 80:2026-2030; and_Cole,S.P.etal.(1984)_Mol.Cell.Biol.62:109-120.)

In addition, techniques developed for the production of "chimeric antibodies", such as the splicing of mouse antibody genes to human antibody genes to obtain a molecule with appropriate antigen specificity and biological activity, can be used (see, e.g., Morrison, S. L. et al. (1984) Proc. Natl. Acad. Sci. USA 81:6851-6855; Neuberger, M. S. et al. (1984) Nature 312:604-608; and Takeda, S. et al. (1985) Nature 314:452-454.). Alternatively, techniques described for the production of single chain antibodies may be adapted, using methods known in the art, to produce PPARGC1β-specific single chain antibodies. Antibodies with related specificity, but of distinct idiotypic composition, may be generated by chain shuffling from random combinatorial immunoglobulin libraries (see, e.g., Burton, D. R. (1991) Proc. Natl. Acad. Sci. USA 88:10134-10137.). Antibodies may also be produced by inducing in vivo production in the lymphocyte population or by screening immunoglobulin libraries or panels of highly specific binding reagents as disclosed in the literature (see, e.g., Orlandi, R. et al. (1989) Proc. Natl. Acad. Sci. USA 86:3833-3837).

Antibody fragments which contain specific binding sites for PPARGC1β may also be generated. For example, such fragments include, but are not limited to, F(ab')₂ fragments produced by pepsin digestion of the antibody molecule and Fab fragments generated by reducing the disulfide bridges of the F(ab')₂ fragments. Alternatively, Fab expression libraries may be constructed to allow rapid and easy identification of monoclonal Fab fragments with the desired specificity (see, e.g., Huse, W. D. et al. (1989) Science 246:1275-1281).

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between PPARGC1β and its specific antibody. A two-site, monoclonal-based immunoassay utilizing monoclonal antibodies reactive to two non-interfering PPARGC1β epitopes is generally used, but a competitive binding assay may also be employed (Pound, supra). Various methods such as Scatchard analysis in conjunction with radioimmunoassay techniques may be used to assess the affinity of antibodies for PPARGC1β. Affinity is expressed as an association constant, Kₐ, which is defined as the molar concentration of PPARGC1β-antibody complex divided by the molar concentrations of free antigen and free antibody under equilibrium conditions. The Kₐ determined for a preparation of polyclonal antibodies, which are heterogeneous in their affinities for multiple PPARGC1β epitopes, represents the average affinity, or avidity, of the antibodies for PPARGC1β. The Kₐ determined for a preparation of monoclonal antibodies, which are monospecific for a particular PPARGC1β epitope, represents a true measure of affinity. High-affinity antibody preparations with Kₐ ranging from about 10⁹ to 10¹² L/mole are preferred for use in immunoassays.

The titer and avidity of polyclonal antibody preparations may be further evaluated to determine the quality and suitability of such preparations for certain downstream applications. For example, a polyclonal antibody preparation containing at least 1-2 mg specific antibody/ml, preferably 5-10 mg specific antibody/ml, is generally employed in procedures requiring precipitation of PPARGC1β-antibody complexes. Procedures for evaluating antibody specificity, titer, and avidity, and guidelines for antibody quality and usage in various applications, are generally available.

Also disclosed are systems (and computer readable media for causing computer systems) to perform a method for detecting and/or identifying that a patient is at risk of thrombosis or an atherothrombotic event or a major bleeding event.

Also disclosed are functional modules, which are defined by computer executable instructions recorded on computer readable media and which cause a computer to perform method steps when executed. The modules are segregated by function for the sake of clarity. However, it should be understood that the modules/systems need not correspond to discreet blocks of code and the described functions can be carried out by the execution of various code portions stored on various media and executed at various times. Furthermore, it should be appreciated that the modules may perform other functions, thus the modules are not limited to having any particular functions or set of functions.

The computer readable storage media #30 can be any available tangible media that can be accessed by a computer. Computer readable storage media includes volatile and nonvolatile, removable and non-removable tangible media implemented in any method or technology for storage of information such as computer readable instructions, data structures, program modules or other data. Computer readable storage media includes, but is not limited to, RAM (random access memory), ROM (read only memory), EPROM (erasable programmable read only memory), EEPROM (electrically erasable programmable read only memory), flash memory or other memory technology, CD-ROM (compact disc read only memory), DVDs (digital versatile disks) or other optical storage media, magnetic cassettes, magnetic tape, magnetic disk storage or other magnetic storage media, other types of volatile and non-volatile memory, and any other tangible medium which can be used to store the desired information and which can accessed by a computer including and any suitable combination of the foregoing.

Computer-readable data embodied on one or more computer-readable storage media may define instructions, for example, as part of one or more programs, that, as a result of being executed by a computer, instruct the computer to perform one or more of the functions described herein, and/or various embodiments, variations and combinations thereof. Such instructions may be written in any of a plurality of programming languages, for example, Java, J#, Visual Basic, C, C#, C++, Fortran, Pascal, Eiffel, Basic, COBOL assembly language, and the like, or any of a variety of combinations thereof. The computer-readable storage media on which such instructions are embodied may reside on one or more of the components of either of a system, or a computer readable storage medium described herein, may be distributed across one or more of such components.

The computer-readable storage media may be transportable such that the instructions stored thereon can be loaded onto any computer resource to implement the aspects of the present invention discussed herein. In addition, it should be appreciated that the instructions stored on the computer-readable medium, described above, are not limited to instructions embodied as part of an application program running on a host computer. Rather, the instructions may be embodied as any type of computer code (e.g., software or microcode) that can be employed to program a computer to implement aspects of the present invention. The computer executable instructions may be written in a suitable computer language or combination of several languages. Basic computational biology methods are known to those of ordinary skill in the art and are described in, for example, Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001).

The functional modules include at minimum a determination system #40, a storage device #30, a comparison module #80, and a display module #110. The functional modules can be executed on one, or multiple, computers, or by using one, or multiple, computer networks. The determination system has computer executable instructions to provide e.g., sequence information in computer readable form.

The determination system #40, can comprise any system for detecting at least one of the allelic variants associated with risk of thrombosis/atherothrombotic event/ major bleeding event.. Standard procedures such as RT-PCR can be used.

Additionally one can determine other factors such as blood pressure, height, weight, circumference of waist, arms, legs, smoking habit, lipid profile, presence of diabetes, fasting glucose, glycosylated haemoglobin, and other newer biomarkers including, but not restricted to, plasma C-reative protein, enzyme activity of lipoprotein-asscocated phospholipase A₂, common carotid intima media thickness and coronary artery calcium. These factors can be used in conjunction with the genetic variant biomarkers.

The information determined in the determination system can be read by the storage device #30. As used herein the "storage device" is intended to include any suitable computing or processing apparatus or other device configured or adapted for storing data or information. Examples of an electronic apparatus suitable for use with the present invention include a stand-alone computing apparatus, data telecommunications networks, including local area networks (LAN), wide area networks (WAN), Internet, Intranet, and Extranet, and local and distributed computer processing systems. Storage devices also include, but are not limited to: magnetic storage media, such as floppy discs, hard disc storage media, magnetic tape, optical storage media such as CD-ROM, DVD, electronic storage media such as RAM, ROM, EPROM, EEPROM and the like, general hard disks and hybrids of these categories such as magnetic/optical storage media. The storage device is adapted or configured for having recorded thereon nucleic acid sequence information. Such information may be provided in digital form that can be transmitted and read electronically, e.g., via the Internet, on diskette, via USB (universal serial bus) or via any other suitable mode of communication.

As used herein, "stored" refers to a process for encoding information on the storage device. Those skilled in the art can readily adopt any of the presently known methods for recording information on known media to generate manufactures comprising information relating to theseallelic variants.

The reference data stored in the storage device to be read by the comparison module is compared, e.g., identity of minor allelic variant for each SNP that is to be detected and correlates with risk.

The "comparison module" #80 can use a variety of available software programs and formats for the comparison operative to compare sequence information data determined in the determination system to reference samples and/or stored reference data. The comparison module is configured to use pattern recognition techniques to compare information from one or more entries to one or more reference data patterns. The comparison module may be configured using existing commercially-available or freely-available software for comparing patterns, and may be optimized for particular data comparisons that are conducted. The comparison module provides computer readable information related to the genotype of the sample.

The comparison module, or any other module of the invention, may include an operating system (e.g., UNIX) on which runs a relational database management system, a World Wide Web application, and a World Wide Web server. World Wide Web application includes the executable code necessary for generation of database language statements (e.g., Structured Query Language (SQL) statements). Generally, the executables will include embedded SQL statements. In addition, the World Wide Web application may include a configuration file which contains pointers and addresses to the various software entities that comprise the server as well as the various external and internal databases which must be accessed to service user requests. The Configuration file also directs requests for server resources to the appropriate hardware--as may be necessary should the server be distributed over two or more separate computers. In one embodiment, the World Wide Web server supports a TCP/IP protocol. Local networks such as this are sometimes referred to as "Intranets." An advantage of such Intranets is that they allow easy communication with public domain databases residing on the World Wide Web (e.g., the GenBank or Swiss Pro World Wide Web site). Thus, in a particular preferred embodiment of the present invention, users can directly access data (via Hypertext links for example) residing on Internet databases using a HTML interface provided by Web browsers and Web servers.

The comparison module provides a computer readable comparison result that can be processed in computer readable form by predefined criteria, or criteria defined by a user, to provide a content based in part on the comparison result that may be stored and output as requested by a user using a display module #110.

The content based on the comparison result is displayed on a computer monitor #120. The content based on the comparison result is displayed through printable media #130, #140. The display module can be any suitable device configured to receive from a computer and display computer readable information to a user. Non-limiting examples include, for example, general-purpose computers such as those based on Intel PENTIUM-type processor, Motorola PowerPC, Sun UltraSPARC, Hewlett-Packard PA-RISC processors, any of a variety of processors available from Advanced Micro Devices (AMD) of Sunnyvale, California, or any other type of processor, visual display devices such as flat panel displays, cathode ray tubes and the like, as well as computer printers of various types.

A World Wide Web browser is used for providing a user interface for display of the content based on the comparison result. It should be understood that other modules can be adapted to have a web browser interface. Through the Web browser, a user may construct requests for retrieving data from the comparison module. Thus, the user will typically point and click to user interface elements such as buttons, pull down menus, scroll bars and the like conventionally employed in graphical user interfaces.

The methods described herein therefore provide for systems (and computer readable media for causing computer systems) to perform methods as described in the Statements of Invention above, for example (a) methods of identifying an individual at risk of thrombosis, an atherothrombotic event, or a major bleeding event, (b) methods of identifying an individual most likely to gain benefit from single anti-platelet therapy in the primary prevention of cardiovascular events, (c) methods of identifying an individual most likely to gain benefit from dual anti-platelet therapy in the secondary prevention of cardiovascular events, (d) methods of identifying an individual likely to suffer a significant bleeding complication when undergoing treatment with drugs which influence haemostasis, (e) methods of identifying an individual likely to suffer thrombotic complications when prescribed COX2 antagonists or PPARy agonists, and (f) methods of identifying an individual suitable for treatment with a higher dosage of anti-platelet therapy.

Systems and computer readable media described herein are merely illustrative for performing methods of diagnosis in an individual, and are not intended to limit the scope of the invention. Variations of the systems and computer readable media described herein are possible.

The modules of the machine, or those used in the computer readable medium, may assume numerous configurations. For example, function may be provided on a single machine or distributed over multiple machines.

### Experimental

The genetic determinants of urinary TxM were investigated. 540 participants in the Hypertension Associated Cardiovascular Disease (HACVD) sub-study of ASCOT gave urine samples at two separate time-points. TxM was measured using liquid chromatography/ tandem mass spectrometry, expressed as pg/mg creatinine to correct for urine concentration. All subjects were genotyped on the CVD50K chip, a cardiovascular-specific chip with >50,000 SNPs. Usual quality control measures were applied. Linear regression analysis was performed, assuming an additive genetic model, adjusting for the covariates; age, sex, smoking habit, diabetes, systolic blood pressure, BMI, HDL, LDL, aspirin and anti-hypertensive regimen.

As expected TxM levels were found to be significantly lower in patients taking aspirin (n=268) *versus* those not on aspirin (n=272) (median [interquartile range]; 324 [211,404] *versus* 716 [460,913] pg/mg, p<0.0001). 21 SNPs in the PPARGC1β gene (See Table 1 below) were significantly associated with higher TxM (beta-values range; +123-+223 pg/mg, p-values range; 0.000004-0.008). Further conditional and haplotype analyses demonstrated that 3 of the 21 variants were independently associated with TxM (rs4235745, rs32582, rs10515638). Most interesting of all was the observation that the association of TxM with the 21 PPARGC1β gene SNPs was almost totally driven by the patients not taking aspirin (beta-values range; +184-+392 pg/mg p-values range; 0.00002-0.06), whilst associations between these same 21 SNPs and TxM amongst aspirinated subjects were in large part not statistically significant (beta-values range; -10-+134 pg/mg p-values range; 0.02-0.97).

The above findings clearly illustrate that genetic variants in PPARGC1β strongly influence thromboxane formation - carriage of one of these SNPs was associated with an increment in TxM of similar magnitude to the decrement in TxM associated with the taking of aspirin. Furthermore since the association was only seen in patients not taking aspirin, it appears that aspirin completely, or in large part, blocks the excess production of thromboxane in carriers of PPARGC1β genetic variants.

As TxM has previously been shown to predict future cardiovascular events such as stroke and myocardial infarction, the same genetic variants in PPARGC1β will serve as a novel genetic biomarker for the identification of individuals at increased risk of thrombosis and atherothrombotic events. The Applicant has also shown an association between the genetic variants and thrombosis and atherothrombotic events, that is independent of the established cardiovascular risk factors: age, sex, blood pressure-lowering treatment regime, lipid-lowering treatment regime, smoking, body mass index, systolic blood pressure, presence of diabetes, and levels of glucose, triglycerides, HDL and total cholesterol.

Furthermore, since carriers of PPARGC1β genetic variants are at increased risk of thrombosis and atherothrombotic events, and aspirin reverses this excess thrombosis risk, these subjects are likely to have an enhanced risk/benefit ratio whilst taking aspirin and/or other anti-platelet drugs.

A further genome wide association study was conducted, using the CNV370 chip seeking further genetic determinants of urinary TxM. The CNV370 chip (Illumina Inc, San Diego, CA, USA) contains 318,000 uniformly spaced and informative single nucleotide polymorphisms (SNPs) and 52,167 CNV markers. The SNPs genotyped on the CNV370-Duo chip were used to impute the genotypes of a further 1,884,210 ungenotyped SNPs using phased haplotype data from the densely genotyped individuals (of European descent) from the HapMap II release 22 CEU reference panel collection, using the MACH program. Similar quality control measures were applied and similar linear regression analyses were performed on the CNV370 data as on the CVD50K data.

Four additional SNPs from 3 genie regions (CNTN4: rs10510230, rs4684343; LZTS1: rs10503687; KCNE4: rs10190010), were significantly associated with TxM levels. See Table 2 for details.

Together the SNPs from the four genie regions (PPARGC1β, CNTN4, LZTS1 and KCNE4 explain a considerable proportion of the variability in TxM in subjects not taking aspirin - in a multivariate analysis PPARGC1β genotype was found to explain 9% of variability, and CNTN4, LZTS1 and KCNE4 genotypes explained an additional 3%, 8%, and 5%, respectively.

### Table 1

**Table 1 lists the SNPs in PPARGC1B which were significantly associated with TxM in all subjects (N=540, P<0.01). Values for the subjects broken down by those taking aspirin, and those not taking aspirin, at time of TxM measurement are also shown. A1 denotes the minor or less frequent allele, A2 the major or more frequent allele. MAF denotes minor allele frequency. BETA values are the increase in TxM (pg/mg creatinine) associated with carriership of 1 minor allele (A1).**

| | | | | | **ALL SUBJECTS (N=540)** | | | | | **TAKING ASPIRIN (N=268)** | | | | | **NOT TAKING ASPIRIN (N=272)** | | | | |
|---|---|---|---|---|---|---|---|---|---|---|---|---|---|---|---|---|---|---|---|
| **SNP** | **CHR** | **BP** | **A1** | **A2** | **A1** | **NMISS** | **BETA** | **P** | **MAF** | **A1** | **NMISS** | **BETA P** | | **MAF** | **A1** | **NMISS** | **BETA** | **P** | **MAF** |
| rs4235745 | 5 | 149171304 | T | C | T | 540 | 167 | 4.3E-06 | 0.30 | T | 268 | 50 | 0.06 | 0.31 | T | 272 | 285 | 2.2E-05 | 0.29 |
| rs32582 | 5 | 149185610 | A | C | A | 540 | 201 | 9.1E-06 | 0.171 | A | 268 | 45 | 0.18 | 0.17 | A | 272 | 353 | 2.1E-05 | 0.17 |
| rs28282 | 5 | 149182399 | G | C | G | 540 | 157 | 1.1E-05 | 0.31 | G | 268 | 49 | 0.07 | 0.31 | G | 272 | 262 | 6.2E-05 | 0.31 |
| rs32581 | 5 | 149185823 | T | G | T | 540 | 159 | 2.1E-05 | 0.28 | T | 268 | 53 | 0.06 | 0.29 | T | 272 | 260 | 1.4E-04 | 0.28 |
| rs32574 | 5 | 149199079 | A | C | A | 539 | 165 | 5.1E-05 | 0.23 | A | 267 | 60 | 0.05 | 0.25 | A | 272 | 273 | 3.1E-04 | 0.21 |
| rs17110447 | 5 | 149173039 | G | A | G | 540 | 149 | 6.0E-05 | 0.28 | G | 268 | 47 | 0.09 | 0.29 | G | 272 | 253 | 2.4E-04 | 0.27 |
| rs2161257 | 5 | 149170190 | G | A | G | 540 | 123 | 2.7E-04 | 0.44 | G | 268 | 52 | 0.05 | 0.45 | G | 272 | 184 | 2.3E-03 | 0.43 |
| rs32579 | 5 | 149191041 | T | C | T | 540 | 130 | 3.7E-04 | 0.30 | T | 268 | 34 | 0.21 | 0.30 | T | 272 | 221 | 9.2E-04 | 0.30 |
| rs10515638 | 5 | 149132724 | T | G | T | 540 | 223 | 7.3E-04 | 0.07 | T | 268 | 75 | 0.12 | 0.07 | T | 272 | 392 | 1.9E-03 | 0.07 |
| rs32586 | 5 | 149181113 | G | A | G | 540 | 173 | 7.6E-04 | 0.121 | G | 268 | 9 | 0.81 | 0.12 | G | 272 | 365 | 2.1E-04 | 0.11 |
| rs32588 | 5 | 149180236 | C | T | C | 540 | 173 | 7.6E-04 | 0.12 | C | 268 | 9 | 0.81 | 0.12 | C | 272 | 365 | 2.1E-04 | 0.11 |
| rs32589 | 5 | 149180082 | A | G | A | 540 | 173 | 7.6E-04 | 0.12 | A | 268 | 9 | 0.81 | 0.12 | A | 272 | 365 | 2.1E-04 | 0.11 |
| rs251464 | 5 | 149176427 | C | G | C | 540 | 125 | 9.4E-04 | 0.26 | C | 268 | 23 | 0.41 | 0.26 | C | 272 | 237 | 9.3E-04 | 0.26 |
| rs251468 | 5 | 149174678 | T | C | T | 540 | 125 | 9.4E-04 | 0.26 | T | 268 | 23 | 0.41 | 0.26 | T | 272 | 237 | 9.3E-04 | 0.26 |
| rs17110375 | 5 | 149104421 | G | A | G | 540 | 208 | 1.3E-03 | 0.07 | G | 268 | 66 | 0.17 | 0.07 | G | 272 | 351 | 3.5E-03 | 0.08 |
| rs251460 | 5 | 149177423 | C | T | C | 540 | 129 | 1.4E-03 | 0.21 | C | 268 | -1 | 0.97 | 0.19 | C | 272 | 257 | 5.2E-04 | 0.22 |
| rs251463 | 5 | 149176522 | A | G | A | 540 | 129 | 1.4E-03 | 0.21 | A | 268 | -1 | 0.97 | 0.19 | A | 272 | 257 | 5.2E-04 | 0.22 |
| rs2052490 | 5 | 149158366 | A | C | A | 540 | 154 | 1.7E-03 | 0.14 | A | 268 | -7 | 0.84 | 0.13 | A | 272 | 327 | 3.7E-04 | 0.14 |
| rs12189417 | 5 | 149168322 | C | G | C | 540 | 152 | 1.8E-03 | 0.14 | C | 268 | -7 | 0.84 | 0.13 | C | 272 | 322 | 4.3E-04 | 0.15 |
| rs32577 | 5 | 149192993 | A | G | A | 540 | 149 | 2.9E-03 | 0.13 | A | 268 | -10 | 0.79 | 0.13 | A | 272 | 313 | 7.8E-04 | 0.13 |
| rs741580 | 5 | 149181863 | G | A | G | 538 | 203 | 8.0E-03 | 0.05 | G | 267 | 134 | 0.02 | 0.05 | G | 271 | 262 | 5.6E-02 | 0.05 |

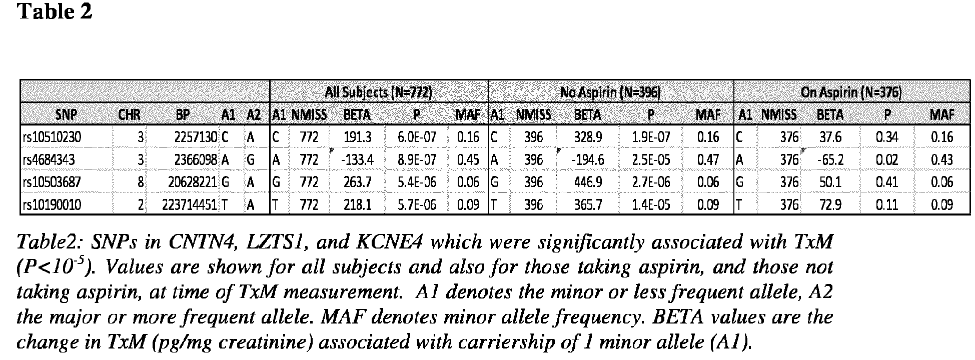

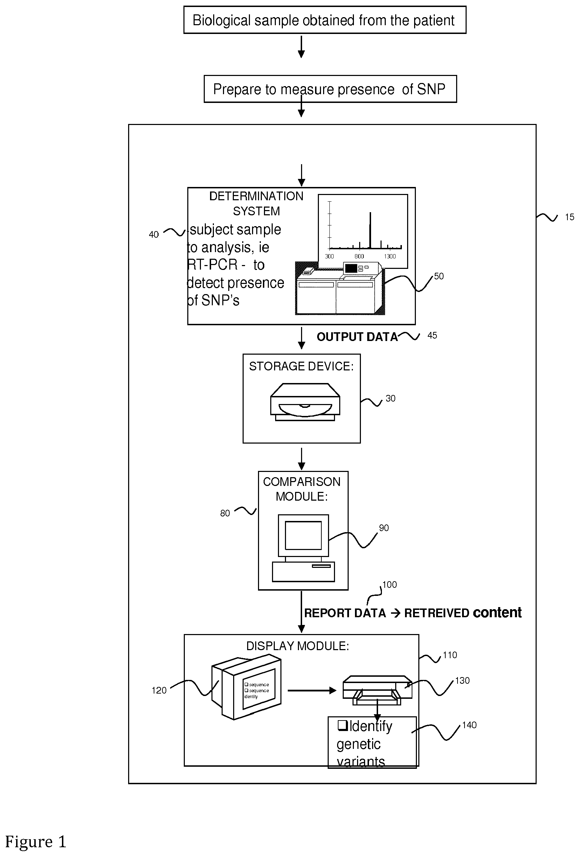

## Claims

1. A method for identification of an individual at increased risk of thrombosis or an atherothrombotic event, comprising a step of assaying a biological sample from the individual for the presence of a minor allelic variant selected from SEQUENCE ID NO's 22 and 25, wherein the presence of the minor allelic variant of SEQUENCE ID NO 22 correlates with the individual being at increased risk of a thrombosis or an atherothrombotic event, and wherein the absence of the minor allelic variant of SEQUENCE ID NO 25 correlates with the individual being at increased risk of a thrombosis or an atherothrombotic event.

2. A method for identification of an individual at increased risk of a major bleeding event selected from cerebral bleeding and gastrointestinal bleeding, comprising a step of assaying a biological sample from the individual for the presence of a minor allelic variant selected from SEQUENCE ID NO's 22 and 25, wherein the absence of a minor allelic variant of SEQUENCE ID NO 22 correlates with the individual being at increased risk of a major bleeding event, and wherein the presence of the minor allelic variant of SEQUENCE ID NO 25 correlates with the individual being at increased risk of a major bleeding event selected from cerebral bleeding and gastrointestinal bleeding.

3. A method of identifying an individual most likely to gain benefit from single anti-platelet therapy in the primary prevention of cardiovascular events, the method comprising a step of assaying a biological sample from the individual for the presence of a minor allelic variant selected from SEQUENCE ID NO's 22 and 25, wherein the presence of a minor allelic variant of SEQUENCE ID NO 22 or absence of the minor allelic variant of SEQUENCE ID NO 25 correlates with the individual being suitable for single anti-platelet therapy.

4. A method of identifying an individual suitable for treatment with a high dosage of anti-platelet therapy, the method comprising a step of assaying a biological sample from the individual for the presence of a minor allelic variant selected from SEQUENCE ID NO's 22 and 25, wherein the presence of a minor allelic variant of SEQUENCE ID NO 22 or absence of the minor allelic variant of SEQUENCE ID NO 25 correlates with the individual being suitable for a high dosage of anti-platelet therapy.

5. A method of identifying an individual most likely to gain benefit from dual anti-platelet therapy in the secondary prevention of cardiovascular events, the method comprising a step of assaying a biological sample from the individual for the presence of a minor allelic variant selected from SEQUENCE ID NO's 22 and 25, wherein the presence of a minor allelic variant of SEQUENCE ID NO 22 or absence of the minor allelic variant of SEQUENCE ID NO 25 correlates with the individual being suitable for dual anti-platelet therapy.

6. A method of identifying an individual likely to suffer a significant bleeding complication when undergoing treatment with drugs which influence haemostasis, the method comprising a step of assaying a biological sample from the individual for the absence of a minor allelic variant selected from SEQUENCE ID NO's 22 and 25, wherein the absence of a minor allelic variant of SEQUENCE ID NO 22 or presence of the minor allelic variant of SEQUENCE ID NO 25 correlates with the individual being likely to suffer a significant bleeding complication when undergoing treatment with a drug that influences haemostasis.

7. A method of identifying an individual likely to suffer a thrombotic complication when prescribed a COX2 antagonist or PPARγ agonist, the method comprising a step of assaying a biological sample from the individual for the presence of a minor allelic variant selected from SEQUENCE ID NO's 22 and 25, wherein the presence of a minor allelic variant of SEQUENCE ID NO 22 or absence of the minor allelic variant of SEQUENCE ID NO 25 correlates with the individual being likely to suffer a thrombotic complication when prescribed a COX2 antagonist or a PPARγ agonist.

8. An assay for identifying a subject as having an increased probability of suffering a thrombotic or atherothrombotic event comprising: (a) identifying at least one allele of a single nucleotide polymorphism selected from the group consisting of SEQUENCE ID NO's 22 and 25 in a biological sample obtained from a subject; and (b) comparing at least one identified allele to a list of corresponding high risk alleles selected from the minor allelic variants of SEQUENCE ID NO 22 and the major allelic variants of SEQUENCE ID NO 25, and if the biological sample comprises one or more of the risk alleles then identifying the subject as having an increased probability of suffering a thrombotic or atherothrombotic event.

9. An assay for identifying a subject having an increased probability of suffering a major bleeding event comprising: (a) identifying at least one allele of a single nucleotide polymorphism selected from the group consisting of SEQUENCE ID NO's 22 and 25 in a biological sample obtained from a subject who is at increased risk of a major bleeding event; and (b) comparing at least one identified allele to a list of corresponding high risk alleles selected from the major allelic variants of SEQUENCE ID NO 22 and the minor allelic variants of SEQUENCE ID NO 25, and if the biological sample comprises one or more of the risk alleles then identifying the subject as having an increased probability of suffering a major bleeding event.

10. An assay for selecting a primary or secondary treatment regimen for a subject at increased risk of thrombosis or atherothrombotic event comprising: (a) subjecting a test sample of the subject to at least one analysis to determine the alleles of a single nucleotide polymorphism selected from the group consisting of SEQUENCE ID NO's 22 and 25; and (b) comparing the at least one identified allele to a list of corresponding high risk alleles selected from the minor allelic variants of SEQUENCE ID NO 22 and the major allelic variants of SEQUENCE ID NO 25, and if the biological sample comprises one or more of the risk alleles then selecting a single anti-platelet therapy as a treatment regime in the primary prevention of a cardiovascular event or dual anti-platelet therapy as a treatment regime in the secondary prevention of a cardiovascular event.

11. An assay for selecting a treatment regime for an individual at increased risk of thrombosis or an atherothrombotic event, the assay comprising: (a) subjecting a test sample of the subject to at least one analysis to determine the alleles of a single nucleotide polymorphism selected from the group consisting of SEQUENCE ID NO's 22 and 25; and (b) comparing the at least one identified allele to a list of corresponding high risk alleles selected from the minor allelic variants of SEQUENCE ID NO 22 and the major allelic variants of SEQUENCE ID NO 25, and if the biological sample comprises one or more of the risk alleles then selecting a high dosage of anti-platelet therapy.

12. An assay for selecting a treatment regime for an individual undergoing treatment with a drug that influences haemostasis: (a) subjecting a test sample of the subject to at least one analysis to determine the alleles of a single nucleotide polymorphism selected from the group consisting of SEQUENCE ID NO's 22 and 25; and (b) comparing the at least one identified allele to a list of corresponding high risk alleles selected from the major allelic variants of SEQUENCE ID NO 22 and the minor allelic variants of SEQUENCE ID NO 25, and if the biological sample comprises one or more of the risk alleles then selecting a treatment that does not include an anti-platelet agent.

## Patentansprüche

1. Verfahren zur Identifizierung eines Patienten, bei dem ein erhöhtes Thromboserisiko oder Risiko eines atherothrombotischen Ereignisses vorliegt, umfassend einen Schritt des Untersuchens einer biologischen Probe von dem Patienten auf die Anwesenheit einer kleineren allelischen Variante, die aus SEQUENZ-ID NR. 22 und 25 ausgewählt ist, wobei die Anwesenheit der kleineren allelischen Variante der SEQUENZ-ID NR. 22 damit korreliert, dass bei dem Patent ein erhöhtes Thromboserisiko oder Risiko eines atherothrombotischen Ereignisses vorliegt, und wobei die Abwesenheit der kleineren allelischen Variante der SEQUENZ-ID NR. 25 damit korreliert, dass bei dem Patient ein erhöhtes Thromboserisiko oder Risiko eines atherothrombotischen Ereignisses vorliegt.

2. Verfahren zur Identifizierung eines Patienten, bei dem ein erhöhtes Risiko eines Ereignisses einer schwereren Blutung vorliegt, das aus einer zerebralen Blutung und einer gastrointestinalen Blutung ausgewählt ist, umfassend einen Schritt des Untersuchens einer biologischen Probe von dem Patienten auf die Anwesenheit einer kleineren allelischen Variante, die aus SEQUENZ-ID NR. 22 und 25 ausgewählt ist, wobei die Abwesenheit einer kleineren allelischen Variante der SEQUENZ-ID NR. 22 damit korreliert, dass der Patent ein erhöhtes Risiko eines Ereignisses einer schwereren Blutung aufweist, und wobei die Anwesenheit der kleineren allelischen Variante der SEQUENZ-ID NR. 25 damit korreliert, dass bei dem Patient ein erhöhtes Ereignisses einer schwereren Blutung vorliegt, das aus einer zerebralen Blutung und einer gastrointestinalen Blutung ausgewählt ist.

3. Verfahren zum Identifizieren eines Patienten, der höchstwahrscheinlich von einer einfachen Thrombozytenhemmtherapie bei der Primärprävention von kardiovaskulären Ereignissen profitieren würde, wobei das Verfahren einen Schritt des Untersuchens einer biologischen Probe von dem Patienten auf die Anwesenheit einer kleineren allelischen Variante umfasst, die aus SEQUENZ-ID NR. 22 und 25 ausgewählt ist, wobei die Anwesenheit einer kleineren allelischen Variante der SEQUENZ-ID NR. 22 oder Abwesenheit der kleineren allelischen Variante der SEQUENZ-ID NR. 25 damit korreliert, dass der Patient für eine einzige Thrombozytenhemmtherapie geeignet ist.

4. Verfahren zum Identifizieren eines Patienten, der für eine Behandlung mit einer hohen Dosierung einer Thrombozytenhemmtherapie geeignet ist, wobei das Verfahren einen Schritt des Untersuchens einer biologischen Probe von dem Patienten auf die Anwesenheit einer kleineren allelischen Variante umfasst, die aus SEQUENZ-ID NR. 22 und 25 ausgewählt ist, wobei die Anwesenheit einer kleineren allelischen Variante der SEQUENZ-ID NR. 22 oder Abwesenheit der kleineren allelischen Variante der SEQUENZ-ID NR. 25 damit korreliert, dass der Patient für eine hohe Dosierung einer Thrombozytenhemmtherapie geeignet ist.

5. Verfahren zum Identifizieren eines Patienten, der höchstwahrscheinlich von einer dualen Thrombozytenhemmtherapie bei der Sekundärprävention von kardiovaskulären Ereignissen profitieren würde, wobei das Verfahren einen Schritt des Untersuchens einer biologischen Probe von dem Patienten auf die Anwesenheit einer kleineren allelischen Variante umfasst, die aus SEQUENZ-ID NR. 22 und 25 ausgewählt ist, wobei die Anwesenheit einer kleineren allelischen Variation der SEQUENZ-ID NR. 22 oder Abwesenheit der kleineren allelischen Variante der SEQUENZ-ID NR. 25 damit korreliert, dass der Patient für eine duale Thrombozytenhemmtherapie geeignet ist.

6. Verfahren zum Identifizieren eines Patienten, bei dem das Erleiden einer erheblichen Blutungskomplikation wahrscheinlich ist, wenn er sich einer Behandlung mit Arzneimitteln unterzieht, die die Hämostase beeinflussen, wobei das Verfahren einen Schritt der Untersuchung einer biologischen Probe von dem Patienten auf die Abwesenheit einer kleineren allelischen Variante umfasst, die aus SEQUENZ-ID NR. 22 und 25 ausgewählt ist, wobei die Abwesenheit einer kleineren allelischen Variante der SEQUENZ-ID NR. 22 oder Anwesenheit der kleineren allelischen Variante der SEQUENZ-ID NR. 25 damit korreliert, dass bei dem Patienten das Erleiden einer erheblichen Blutungskomplikation wahrscheinlich ist, wenn er sich einer Behandlung mit einem Arzneimittel unterzieht, das die Hämostase beeinflusst.

7. Verfahren zum Identifizieren eines Patienten, bei dem das Erleiden einer thrombotischen Komplikation wahrscheinlich ist, wenn ihm ein COX-2-Antagonist oder PPARγ-Agonist verschrieben wird, wobei das Verfahren einen Schritt des Untersuchens einer biologischen Probe von dem Patienten auf die Anwesenheit einer kleineren allelischen Variante umfasst, die aus SEQUENZ-ID NR. 22 und 25 ausgewählt ist, wobei die Anwesenheit einer kleineren allelischen Variante der SEQUENZ-ID NR. 22 oder Abwesenheit der kleineren allelischen Variante der SEQUENZ-ID NR. 25 damit korreliert, dass bei dem Patienten das Erleiden einer thrombotischen Komplikation wahrscheinlich ist, wenn ihm ein COX-2-Antagonist oder PPARγ-Agonist verschrieben wird.

8. Assay zum Identifizieren eines Patienten, bei dem eine erhöhte Wahrscheinlichkeit des Erleidens eines thrombotischen oder atherothrombotischen Ereignisses vorliegt, der Folgendes umfasst: (a) Identifizieren von zumindest einem Allel eines Einzelnukleotid-Polymorphismus, das aus der Gruppe ausgewählt ist, die aus SEQUENZ-ID NR. 22 und 25 besteht, in einer biologischen Probe, die von einem Patienten erhalten wurde; und (b) Vergleichen von zumindest einem identifizierten Allel mit einer Liste von entsprechenden Allelen mit hohem Risiko, die aus den kleineren allelischen Varianten der SEQUENZ-ID NR. 22 und der größeren allelischen Varianten der SEQUENZ-ID NR. 25 ausgewählt sind, und Identifizieren, dass bei dem Patienten eine erhöhte Wahrscheinlichkeit des Erleidens eines thrombotischen oder atherothrombotischen Ereignisses vorliegt, wenn die biologische Probe ein oder mehrere der Risikoallele umfasst.

9. Assay zum Identifizieren eines Patienten, bei dem eine erhöhte Wahrscheinlichkeit des Erleidens eines schwerwiegenden Blutungsereignis vorliegt, der Folgendes umfasst: (a) Identifizieren von zumindest einem Allel eines Einzelnukleotid-Polymorphismus, das aus der Gruppe ausgewählt ist, die aus SEQUENZ-ID NR. 22 und 25 besteht, in einer biologischen Probe, die von einem Patienten erhalten wurde, bei dem ein erhöhtes Risiko eines schwerwiegenden Blutungsereignisses vorliegt; und (b) Vergleichen von zumindest einem identifizierten Allel mit einer Liste von entsprechenden Allelen mit hohem Risiko, die aus den größeren allelischen Varianten der SEQUENZ-ID NR. 22 und den kleineren allelischen Varianten der SEQUENZ-ID NR. 25 ausgewählt sind, und Identifizieren, dass bei dem Patienten eine erhöhte Wahrscheinlichkeit des Erleidens eines schwerwiegenden Blutungsereignisses vorliegt, wenn die biologische Probe ein oder mehrere der Risikoallele umfasst.

10. Assay zum Auswählen eines Primär- oder Sekundärbehandlungsregimes für einen Patienten, bei dem ein erhöhtes Thromboserisiko oder Risiko eines atherothrombotisches Ereignisses vorliegt, der Folgendes umfasst: (a) Unterziehen einer Testprobe des Patienten zumindest einer Analyse, um die Allele eines Einzelnukleotid-Polymorphismus zu bestimmen, die aus der Gruppe ausgewählt sind, die aus SEQUENZ-ID NR. 22 und 25 besteht; und (b) Vergleichen des zumindest einen identifizierten Allels mit einer Liste von entsprechenden Allelen mit hohem Risiko, die aus den kleineren allelischen Varianten der SEQUENZ-ID NR. 22 und den größeren allelischen Varianten der SEQUENZ-ID NR. 25 ausgewählt sind, und Auswählen einer einfachen Thrombozytenhemmtherapie als ein Behandlungsregime bei der Primärprävention eines kardiovaskulären Ereignisses oder einer dualen Thrombozytenhemmtherapie als ein Behandlungsregime bei der Sekundärprävention eines kardiovaskulären Ereignisses, wenn die biologische Probe ein oder mehrere der Risikoallele umfasst.

11. Assay zum Auswählen eines Behandlungsregimes für einen Patienten, bei dem ein erhöhtes Risiko für Thrombose oder ein atherothrombotisches Ereignis vorliegt, wobei der Assay Folgendes umfasst: (a) Unterziehen einer Testprobe des Patienten zumindest einer Analyse, um die Allele eines Einzelnukleotid-Polymorphismus zu bestimmen, die aus der Gruppe ausgewählt sind, die aus SEQUENZ-ID NR. 22 und 25 besteht; und (b) Vergleichen des zumindest einen identifizierten Allels mit einer Liste von entsprechenden Allelen mit hohem Risiko, die aus den kleineren allelischen Varianten der SEQUENZ-ID NR. 22 und den größeren allelischen Varianten der SEQUENZ-ID NR. 25 ausgewählt sind, und Auswählen einer hohen Dosierung einer Thrombozytenhemmtherapie, wenn die biologische Probe ein oder mehrere der Risikoallele umfasst.

12. Assay zum Auswählen eines Behandlungsregimes für einen Patienten, der mit einem Arzneimittel behandelt wird, das Hämostase beeinflusst: (a) Unterziehen einer Testprobe des Patienten zumindest einer Analyse, um die Allele eines Einzelnukleotid-Polymorphismus zu bestimmen, die aus der Gruppe ausgewählt sind, die aus SEQUENZ-ID NR. 22 und 25 besteht; und (b) Vergleichen des zumindest einen identifizierten Allels mit einer Liste von entsprechenden Allelen mit hohem Risiko, die aus den größeren allelischen Varianten der SEQUENZ-ID NR. 22 und den kleineren allelischen Varianten der SEQUENZ-ID NR. 25 ausgewählt sind, und Auswählen einer Behandlung, die keinen Thrombozytenhemmer beinhaltet, wenn die biologische Probe ein oder mehrere der Risikoallele umfasst.

## Revendications

1. Procédé d'identification d'un individu présentant un risque accru de thrombose ou d'un événement athérothrombotique, comprenant une étape consistant à mettre à l'essai un échantillon biologique provenant de l'individu quant à la présence d'un variant allélique mineur choisi parmi SEQUENCE ID N° 22 et 25, dans lequel la présence du variant allélique mineur de SEQUENCE ID N° 22 se corrèle au fait que l'individu présente un risque accru d'une thrombose ou d'un événement athérothrombotique, et dans lequel l'absence du variant allélique mineur de SEQUENCE ID N° 25 se corrèle au fait que l'individu présente un risque accru d'une thrombose ou d'un événement athérothrombotique.

2. Procédé d'identification d'un individu présentant un risque accru d'un événement d'hémorragie majeure choisi parmi une hémorragie cérébrale et une hémorragie gastro-intestinale, comprenant une étape consistant à mettre à l'essai un échantillon biologique provenant de l'individu quant à la présence d'un variant allélique mineur choisi parmi SEQUENCE ID N° 22 et 25, dans lequel l'absence d'un variant allélique mineur de SEQUENCE ID N° 22 se corrèle au fait que l'individu présente un risque accru d'un événement d'hémorragie majeure, et dans lequel la présence du variant allélique mineur de SEQUENCE ID N° 25 se corrèle au fait que l'individu présente un risque accru d'un événement d'hémorragie majeure choisi parmi une hémorragie cérébrale et une hémorragie gastro-intestinale.

3. Procédé d'identification d'un individu le plus susceptible de tirer profit d'une thérapie antiplaquettaire unique dans la prévention primaire d'événements cardiovasculaires, le procédé comprenant une étape consistant à mettre à l'essai un échantillon biologique provenant de l'individu quant à la présence d'un variant allélique mineur choisi parmi SEQUENCE ID N° 22 et 25, dans lequel la présence d'un variant allélique mineur de SEQUENCE ID N° 22 ou l'absence du variant allélique mineur de SEQUENCE ID N° 25 se corrèle au fait que l'individu convienne à une thérapie antiplaquettaire unique.

4. Procédé d'identification d'un individu convenant au traitement avec une posologie élevée de thérapie antiplaquettaire, le procédé comprenant une étape consistant à mettre à l'essai un échantillon biologique provenant de l'individu quant à la présence d'un variant allélique mineur choisi parmi SEQUENCE ID N° 22 et 25, dans lequel la présence d'un variant allélique mineur de SEQUENCE ID N° 22 ou l'absence du variant allélique mineur de SEQUENCE ID N° 25 se corrèle au fait que l'individu convienne à une posologie élevée de thérapie antiplaquettaire.

5. Procédé d'identification d'un individu le plus susceptible de tirer profit d'une thérapie antiplaquettaire double dans la prévention secondaire d'événements cardiovasculaires, le procédé comprenant une étape consistant à mettre à l'essai un échantillon biologique provenant de l'individu quant à la présence d'un variant allélique mineur choisi parmi SEQUENCE ID N° 22 et 25, dans lequel la présence d'un variant allélique mineur de SEQUENCE ID N° 22 ou l'absence du variant allélique mineur de SEQUENCE ID N° 25 se corrèle au fait que l'individu convienne à une thérapie antiplaquettaire double.

6. Procédé d'identification d'un individu susceptible de souffrir d'une complication d'hémorragie significative lorsqu'il subit un traitement avec des médicaments qui influencent l'hémostase, le procédé comprenant une étape consistant à mettre à l'essai un échantillon biologique provenant de l'individu quant à l'absence d'un variant allélique mineur choisi parmi SEQUENCE ID N° 22 et 25, dans lequel l'absence d'un variant allélique mineur de SEQUENCE ID N° 22 ou la présence du variant allélique mineur de SEQUENCE ID N° 25 se corrèle au fait que l'individu soit susceptible de souffrir d'une complication d'hémorragie significative lorsqu'il subit un traitement avec un médicament qui influence l'hémostase.

7. Procédé d'identification d'un individu susceptible de souffrir d'une complication thrombotique lorsqu'il se voit prescrit un antagoniste COX2 ou un agoniste PPARγ, le procédé comprenant une étape consistant à mettre à l'essai un échantillon biologique provenant de l'individu quant à la présence d'un variant allélique mineur choisi parmi SEQUENCE ID N° 22 et 25, dans lequel la présence d'un variant allélique mineur de SEQUENCE ID N° 22 ou l'absence du variant allélique mineur de SEQUENCE ID N° 25 se corrèle au fait que l'individu soit susceptible de souffrir d'une complication thrombotique lorsqu'il se voit prescrit un antagoniste COX2 ou un agoniste PPARγ.

8. Essai d'identification d'un sujet comme ayant une probabilité accrue de souffrir d'un événement thrombotique ou athérothrombotique comprenant : (a) l'identification d'au moins un allèle d'un polymorphisme mononucléotidique choisi dans le groupe consistant en SEQUENCE ID N° 22 et 25 dans un échantillon biologique obtenu auprès d'un sujet ; et (b) la comparaison d'au moins un allèle identifié à une liste d'allèles de haut risque correspondants choisis parmi les variants alléliques mineurs de SEQUENCE ID N° 22 et les variants alléliques majeurs de SEQUENCE ID N° 25, et si l'échantillon biologique comprend un ou plusieurs des allèles à risque, alors l'identification du sujet comme ayant une probabilité accrue de souffrir d'un événement thrombotique ou athérothrombotique.

9. Essai d'identification d'un sujet ayant une probabilité accrue de souffrir d'un événement d'hémorragie majeure comprenant : (a) l'identification d'au moins un allèle d'un polymorphisme mononucléotidique choisi dans le groupe consistant en SEQUENCE ID N° 22 et 25 dans un échantillon biologique obtenu auprès d'un sujet qui présente un risque accru d'un événement d'hémorragie majeure ; et (b) la comparaison d'au moins un allèle identifié à une liste d'allèles à haut risque correspondants choisis parmi les variants alléliques majeurs de SEQUENCE ID N° 22 et les variants alléliques mineurs de SEQUENCE ID N° 25, et si l'échantillon biologique comprend un ou plusieurs des allèles à risque, alors l'identification du sujet comme ayant une probabilité accrue de souffrir d'un événement d'hémorragie majeure.

10. Essai de sélection d'un régime de traitement primaire ou secondaire pour un sujet présentant un risque accru de thrombose ou d'événement athérothrombotique comprenant : (a) la soumission d'un échantillon de test du sujet à au moins une analyse pour déterminer les allèles d'un polymorphisme mononucléotidique choisi dans le groupe consistant en SEQUENCE ID N° 22 et 25 ; et (b) la comparaison de l'au moins un allèle identifié à une liste d'allèles à haut risque correspondants choisis parmi les variants alléliques mineurs de SEQUENCE ID N° 22 et les variants alléliques majeurs de SEQUENCE ID N° 25, et si l'échantillon biologique comprend un ou plusieurs des allèles à risque, alors la sélection d'une thérapie antiplaquettaire unique comme régime de traitement dans la prévention primaire d'un événement cardiovasculaire ou une thérapie antiplaquettaire double comme régime de traitement dans la prévention secondaire d'un événement cardiovasculaire.

11. Essai de sélection d'un régime de traitement pour un individu présentant un risque accru de thrombose ou d'un événement athérothrombotique, l'essai comprenant : (a) la soumission d'un échantillon de test du sujet à au moins une analyse pour déterminer les allèles d'un polymorphisme mononucléotidique choisi dans le groupe consistant en SEQUENCE ID N° 22 et 25 ; et (b) la comparaison de l'au moins un allèle identifié à une liste d'allèles à haut risque correspondants choisis parmi les variants alléliques mineurs de SEQUENCE ID N° 22 et les variants alléliques majeurs de SEQUENCE ID N° 25, et si l'échantillon biologique comprend un ou plusieurs des allèles à risque, alors la sélection d'une posologie élevée de thérapie antiplaquettaire.

12. Essai de sélection d'un régime de traitement pour un individu subissant un traitement avec un médicament qui influence l'hémostase : (a) la soumission d'un échantillon de test du sujet à au moins une analyse pour déterminer les allèles d'un polymorphisme mononucléotidique choisi dans le groupe consistant en SEQUENCE ID N° 22 et 25 ; et (b) la comparaison de l'au moins un allèle identifié à une liste d'allèles à haut risque correspondants choisis parmi les variants alléliques majeurs de SEQUENCE ID N° 22 et les variants alléliques mineurs de SEQUENCE ID N° 25, et si l'échantillon biologique comprend un ou plusieurs des allèles à risque, alors la sélection d'un traitement qui n'inclut pas un agent antiplaquettaire.


### SEQUENCE LISTING

<110> Royal College of Surgeons in Ireland
<120> Identification of Thrombosis or Bleeding Risk in an Individual with and without Anti-platelet Therapy
<130> P10685PC00
<150> EP11166142.7
   <151> 2011-05-14
<160> 25
<170> PatentIn version 3.3
<210> 1
   <211> 61
   <212> DNA
   <213> Homo sapiens
<220>
   <221> variation
   <222> (31)..(31)
<300>
   <308> rs4235745
   <309> 2009-01-17
   <313> (1)..(61)
<300>
   <308> dbSNP/rs4235745
   <309> 2009-01-17
   <313> (1)..(61)
<400> 1
   agctgaatct tagtaaataa acaactaagt yggtgggagc agaccagctg aggctctcag      60
   g                                                                      61
<210> 2
   <211> 61
   <212> DNA
   <213> Homo sapiens
<220>
   <221> variation
   <222> (31)..(31)
<300>
   <308> dbSNP/rs32582
   <309> 2009-01-17
   <313> (1)..(61)
<400> 2
   ccaccactgt catcatcatc atcgtcatgg magaaatcgt gtaacagtag cagcactagc      60
   a                                                                      61
<210> 3
   <211> 61
   <212> DNA
   <213> Homo sapiens
<220>
   <221> variation
   <222> (31)..(31)
<300>
   <308> dbSNP/rs28282
   <309> 2009-01-17
   <313> (1)..(61)
<400> 3
   actgaatgaa tccttcgaca cgcagctctc stgatgcctc ctcctaaggc agttggtcac      60
   t                                                                      61
<210> 4
   <211> 61
   <212> DNA
   <213> Homo sapiens
<220>
   <221> variation
   <222> (31)..(31)
<300>
   <308> dbSNP/rs32581
   <309> 2009-01-17
   <313> (1)..(61)
<400> 4
   gagaaagcta acttgcctga gagcccagag ktgctaagta tgggacctgg ggtacagccc 60
   c 61
<210> 5
   <211> 61
   <212> DNA
   <213> Homo sapiens
<220>
   <221> variation
   <222> (31)..(31)
<300>
   <308> dbSNP/rs32574
   <309> 2009-01-17
   <313> (1)..(61)
<400> 5
   gtcacccaac agggaggcgg tggggccaag mttcaaactc aagctgtctg actcacaagc 60
   c 61
<210> 6
   <211> 61
   <212> DNA
   <213> Homo sapiens
<220>
   <221> variation
   <222> (31)..(31)
<300>
   <308> dbSNP/rs17110447
   <309> 2008-12-18
   <313> (1)..(61)
<400> 6
   cttgcctcag atcatcaaat acaacttctt rtcagtgaag gaggcaccaa gctggcagtc 60
   g 61
<210> 7
   <211> 61
   <212> DNA
   <213> Homo sapiens
<220>
   <221> variation
   <222> (31)..(31)
<300>
   <308> dbSNP/rs2161257
   <309> 2009-01-17
   <313> (1)..(61)
<400> 7
   gggtgggggg cctagctgac ctcggtgctg raggagggga gtctgggctc tgcacagcca      60
   c                                                                      61
<210> 8
   <211> 61
   <212> DNA
   <213> Homo sapiens
<220>
   <221> variation
   <222> (31)..(31)
<300>
   <308> dbSNP/rs32579
   <309> 2009-01-17
   <313> (1)..(61)
<400> 8
   agggagccca ttggagggtt taaagctctg ycatagacat ccagggtcaa agaacatccc      60
   t                                                                      61
<210> 9
   <211> 61
   <212> DNA
   <213> Homo sapiens
<220>
   <221> variation
   <222> (31)..(31)
<300>
   <308> dbSNP/rs10515638
   <309> 2009-01-17
   <313> (1)..(61)
<400> 9
   gttgtactgt gaacatacct ggtgcttaag ktaaaggcgc tggctgtggt ggcgttgcag      60
   g                                                                      61
<210> 10
   <211> 61
   <212> DNA
   <213> Homo sapiens
<220>
   <221> variation
   <222> (31)..(31)
<300>
   <308> dbSNP/rs32586
   <309> 2009-01-17
   <313> (1)..(61)
<400> 10
   gggcatgtaa tggctagcat ttcctgtaag raggcctctt acggaataat atgatgtctg      60
   g                                                                      61
<210> 11
   <211> 61
   <212> DNA
   <213> Homo sapiens
<220>
   <221> variation
   <222> (31)..(31)
<300>
   <308> dbSNP/rs32588
   <309> 2009-01-17
   <313> (1)..(61)
<400> 11
   ggagcaactc tatgctgact ttccagaact ygacctctcc cagctggatg ccagcgactt      60
   t                                                                      61
<210> 12
   <211> 61
   <212> DNA
   <213> Homo sapiens
<220>
   <221> variation
   <222> (31)..(31)
<300>
   <308> dbSNP/rs32589
   <309> 2009-01-17
   <313> (1)..(61)
<400> 12
   ctttccattt cttggtcatt tggcaaaatc rggccttggc cacgggtcca gattagctgc      60
   a                                                                      61
<210> 13
   <211> 61
   <212> DNA
   <213> Homo sapiens
<220>
   <221> variation
   <222> (31)..(31)
<300>
   <308> dbSNP/rs251464
   <309> 2009-01-17
   <313> (1)..(61)
<400> 13
   ctggttctaa agctgtttct aagaagcaag scctacaaag gtggctgctc tgagttactg      60
   g                                                                      61
<210> 14
   <211> 61
   <212> DNA
   <213> Homo sapiens
<220>
   <221> variation
   <222> (31)..(31)
<300>
   <308> dbSNP/rs251468
   <309> 2009-01-17
   <313> (1)..(61)
<400> 14
   cacgttgagg ggccagagag gccctacaca ygggccgtac aaacagtgtg tttggtccca      60
   g                                                                      61
<210> 15
   <211> 61
   <212> DNA
   <213> Homo sapiens
<220>
   <221> variation
   <222> (31)..(31)
<300>
   <308> dbSNP/rs17110375
   <309> 2009-01-17
   <313> (1)..(61)
<400> 15
   atttataaag tttgcatatt gttagcatac rtaattcctt gatgaagcca gatgaatgag      60
   t                                                                      61
<210> 16
   <211> 61
   <212> DNA
   <213> Homo sapiens
<220>
   <221> variation
   <222> (31)..(31)
<300>
   <308> dbSNP/rs251460
   <309> 2009-01-17
   <313> (1)..(61)
<400> 16
   tgagtcttat ataacacagt gcctggctca yagtagcatt gaacatgccc actagaatgg 60
   c 61
<210> 17
   <211> 61
   <212> DNA
   <213> Homo sapiens
<220>
   <221> variation
   <222> (31)..(31)
<300>
   <308> dbSNP/rs251463
   <309> 2009-01-17
   <313> (1)..(61)
<400> 17
   gatcttattg ctgggggctt tgctggcctc rgaggcccca gctgcctagc aacccgtgtc 60
   t 61
<210> 18
   <211> 61
   <212> DNA
   <213> Homo sapiens
<220>
   <221> variation
   <222> (31)..(31)
<300>
   <308> dbSNP/rs2052490
   <309> 2009-01-17
   <313> (1)..(61)
<400> 18
   agaaggctga ggcctagggc catggggtgg mctgattata atcacatagc tccagccaat 60
   g 61
<210> 19
   <211> 61
   <212> DNA
   <213> Homo sapiens
<220>
   <221> variation
   <222> (31)..(31)
<300>
   <308> dbSNP/rs12189417
   <309> 2009-01-17
   <313> (1)..(61)
<400> 19
   gtagaggggc ctggaaccag gaaagaaact saactctcag cgtttttgcc ccccacagac      60
   t                                                                      61
<210> 20
   <211> 61
   <212> DNA
   <213> Homo sapiens
<220>
   <221> variation
   <222> (31)..(31)
<300>
   <308> dbSNP/rs32577
   <309> 2009-01-17
   <313> (1)..(61)
<400> 20
   caaagacagt caggcctccc ctggtcgccc rtcctcggtg gaggaggtaa ggatcgcagc      60
   t                                                                      61
<210> 21
   <211> 61
   <212> DNA
   <213> Homo sapiens
<220>
   <221> variation
   <222> (31)..(31)
<300>
   <308> dbSNP/rs741580
   <309> 2009-01-17
   <313> (1)..(61)
<400> 21
   cctccatttt cttgcttgaa caatggggac rattcctttg ctatattgtt cctttcctct      60
   t                                                                      61
<210> 22
   <211> 52
   <212> DNA
   <213> Homo sapiens
<220>
   <221> variation
   <222> (27)..(27)
<300>
   <308> dbSNP/rs10510230
   <309> 2003-11-13
   <313> (1)..(49)
<400> 22
   ttccaaatat accaacagca gatatcmaat gaccattact tcatactaga ag 52
<210> 23
   <211> 52
   <212> DNA
   <213> Homo sapiens
<220>
   <221> variation
   <222> (27)..(27)
<300>
   <308> dbSNP/rs10503687
   <309> 2007-04-20
   <313> (1)..(49)
<400> 23
   acctatgcat cattctaact ggtcacrtcc cactgtagcc aacctcagaa gt 52
<210> 24
   <211> 52
   <212> DNA
   <213> Homo sapiens
<220>
   <221> variation
   <222> (27)..(27)
<300>
   <308> dbSNP/10190010
   <309> 2003-11-05
   <313> (1)..(49)
<400> 24
   caaacaccat gcctagtgcc tcagacwgta cctggaactt ggtaggaact ta 52
<210> 25
   <211> 52
   <212> DNA
   <213> Homo sapiens
<220>
   <221> variation
   <222> (27)..(27)
<300>
   <308> dbSNP/4684343
   <309> 2003-02-12
   <313> (1)..(49)
<400> 25
   gtacacaata acccttctaa ttgcacrtga aatgaaggac tgtaaacttc at 52